Europäisches Patentamt

European Patent Office

Office européen des brevets

(11) Veröffentlichungsnummer: **0 004 277**
**B1**

(12) ## EUROPÄISCHE PATENTSCHRIFT

(21) Anmeldenummer: **79100389.0**

(22) Anmeldetag: **12.02.79**

(51) Int. Cl.³: **C 07 C 139/14**
**C 07 C 143/38**

(54) Verfahren zur Erhöhung der Lagerstabilität von feindispersen aromatischen isocyanatosulfonsäuren, sowie die entsprechenden stabilisierten Zubereitungen

(30) Priorität: **22.02.78 DE 2807458**

(43) Veröffentlichungstag der Anmeldung:
**03.10.79 Patentblatt 79/20**

(45) Bekanntmachung des Hinweises auf die Patenterteilung:
**10.12.80 Patentblatt 80/25**

(84) Benannte Vertragsstaaten:
**BE CH DE FR GB IT**

(56) Entgegenhaltungen:
**DE - A - 2 640 103**
**DE - A - 2 650 172**

(73) Patentinhaber: **BAYER AG**
**Zentralbereich Patente, Marken und Lizenzen**
**D - 5090 Leverkusen 1, Bayerwerk (DE)**

(72) Erfinder: **Dietrich, Dieter, Dr.**
**Ludwig-Girtlerstrasse 1**
**D - 5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Erhöhung der Lagerstabilität von feindispersen aromatischen Isocyanatosulfonsäuren, sowie die entsprechenden stabilisierten Zubereitungen

Sulfonsäuren aromatischer Di- bzw. Polyisocyanate sind bekannt. Sie werden in einfacher Weise durch Umsetzung entsprechender aromatischer di- bzw. Polyisocyanate mit Sulfonierungsmitteln wie Schwefeltrioxid, Addukten des Schwefeltrioxids, Oleum, Chlorsulfonsäure oder Schwefelsäure erhalten (z.B. DT-OS 2 227 111, DT-OS 2 359 615, US-PS 3 826 769).

Je nach verwendetem Isocyanat und dem Grad der Sulfonierung können feste, harzartige oder pulverförmige Sulfinierungsprodukte oder Lösungen der sulfonierten Isocyanate in unverändertem Ausgangsisocyanat erhalten werden.

Während die Handhabung flüssiger sulfonierter Polyisocyanate keinerlei Schweirigkeiten mit sich bringt, ergeben sich bei der Herstellung, der Lagerung und der Verwendung fester pulverförmiger Isocyanatosulfonsäuren erhebliche Probleme.

So fallen diese Produkte bei der Herstellung häufig so feinteilig an, daß die Abtrennung und Reinigung vom flüssigen Reaktionsmedium Schwierigkeiten bereitet. Die getrockneten Produkte stauben beim Ab- und Umfüllen. Vor allem aber sind so hergestellte Trockenpulver nicht lagerstabil. Während der Lagerung steigen der Schmelz- bzw. Zersetzungspunkt an und die Produkte werden in organischen Lösungsmitteln sowie in den bei der Herstellung von Polyurethanen üblicherweise verwendeten Polyestern, Polyäthern und Polyolen zunehmend unlöslich.

Zwar besteht das Problem einer ausreichenden Lagerstabilität bei Polyisocyanaten ganz allgemein und ist daher dem Fachmann im Prinzip vertraut; bei den festen pulverförmigen Isocyanatosulfonsäuren tritt die Beeinträchtigung der Qualität nach der Herstellung jedoch in so kurzer Zeit, z.B. bereits nach wenigen Tagen, ein, daß die Herstellung technisch brauchbarer Polyadditionsprodukte dadurch ganz erheblich erschwert, wenn nicht unmöglich gemacht wird.

Andererseits besteht ein technische-wirtschaftliches Bedürfnis neben oder anstelle der bekannten üblichen Di- und Polyisocyanate auch Polyisocyanatosulfonsäuren im Rahmen der Polyadditionschemie einzusetzen, da diese Isocyanate hervorragende Ausgangsprodukte zur Herstellung hydrophiler, insbesondere wasserdispergierbarer Polyurethane darstellen und da sie außerdem vom physiologischen bzw. gewerbehygienischen Gesichtspunkt besonders vorteilhaft erscheinen, da sie keinen Dampfdruck aufweisen und beim Abbau wasserlösliche Amino-sulfonsäuren liefern. Bisher wurde bei der Herstellung von Polyurethanen auf der Basis von sulfoniertem Toluylendiisocyanat so verfahren, daß man entweder anstelle des reinen Diisocyanats das daraus hergestellte Präpolymer sulfonierte (US-PS 3 826 769). oder man stellte die Isocyanatosulfonsäure erst kurz vor der Weiterverarbeitung zum Polyurethan her (US-PS 3 826 769). Die erste Methode hat den Nachteil, daß man in der Verwendung des Sulfonierungsmittels beschränkt ist, da z.B. Schwefeltrioxid bei der Einwirkung auf Polyätherpräpolymere Zersetzungserscheinungen hervorruft. Nimmt man zur Sulfonierung Schwefelsäure, so ist eine gleichzeitige Kettenverlängerung unter Ausbildung von Harnstoffgruppen unvermeidlich. auch lassen sich nach dieser Methode nur ganz oder teilweise freie Isocyanate sulfonieren, nicht dagegen in der Form von Urethanen vorliegende Produkte. Bei einem NCO-Präpolymer werden also nur die endständigen Isocyanateinheiten sulfoniert. Die zweite Methode ist technisch nicht zu praktizieren, da dem Hersteller eines Polyurethans nicht zuzumuten ist, vorher eine Isocyanatsulfonierung durchzuführen.

Es ist auch vorgeschlagen worden (US-Patentschrift 3 826 769), die Isocyanatosulfonsäuren unmittelbar nach ihrer Herstellung in einem organischen Lösungsmittel, z.B. Aceton, zu lösen und als Lösung zur Anwendung zu bringen, Auch diese Methode ist in der technischen Praxis nicht realisierbar, da z.B. eine Lösung von sulfoniertem Toluylendiisocyanat in Aceton höchstens wenige Stunden stabil ist und danach rasch Trübung und Niederschlagsbildung eintritt.

Es ist daher nicht überraschend, daß feste Isocyanatosulfonsäuren bisher keinen Eingang in die Technik gefunden haben.

Es bestand daher die Aufgabe, Isocyanatosulfonsäuren so herzustellen, bzw. so zu stabilisieren, daß sie problemlos zu lagern und anzuwenden sind und ihre Löslichkeit in organischen Medien auch nach längerer Lagerung erhalten bleibt.

Die vorliegende Erfindung weist einen Weg zur Lösung dieser Aufgabe. Es wurde nämlich überraschenderweise gefunden, daß sich Isocyanatosulfonsäuren durch Zusatz eines weitgehend unpolaren, inerten Feststoffs mit einem zwischen 27 und 250°C liegenden Schmelzpunkt, der vorzugsweise in geschmolzener Form kein Lösungsmittel für die aromatischen Isocyanatosulfonsäuren darstellt, also als Suspendiermittel wirkt, wirksam stabilisieren lassen. Bereits ein Zusatz von ca. 5 Gew.-% des festen Suspendiermittels reicht zur Stabilisierung aus, wobei ein nicht staubendes Pulver erhalten wird.

Gegenstand der vorliegenden Erfindung ist somit ein Verfahren zur Erhöhung der Lagerstabilität von feindispersen,, festen, durch Sulfonierung von aromatischen Isocyanaten erhaltenen, aromatischen Isocyanatosulfonsäuren, dadurch gekennzeichnet, daß

a) man die Sulfonierung der aromatischen Isocyanate in Gegenwart einer inerten Flüssigkeit durchführt, welche für die aromatischen Isocyanatosulfonsäuren kein Lösungsmittel ist, und welche entweder aus einer Schmelze eines zwischen 27 und 250°C schmelzenden inerten organischen Feststoffs oder aus einer mindestens 10-gew.-%igen Lösung eines derartigen Feststoffs in einem inerten Lösungsmittel besteht, wobei man nach Beendigung der Sulfonierungsreaktion den besagten Feststoff zumindest teilweise in dem Sulfonierungsprodukt beläßt, oder

b) man in Abwesenheit einer Flüssigkeit der unter a) definierten Art hergestellte, feste, feindisperse, aromatische Isocyanatosulfonsäuren mit einer derartigen Flüssigkeit möglichst bald nach der Herstellung vermengt.

Gegenstand der vorliegenden Erfindung sind auch lagerstabile gegebenenfalls in Form eines Feuchtpulvers oder einer Paste vorliegende Gemische enthaltend

a) 40—95 Gew.-% an festen, feindispersen, aromatischen Isocyanatosulfonsäuren und
b) 60—5 Gew.-% eines unpolaren, gegenüber Isocyanatgruppen und Sulfonsäuregruppen inerten, in geschmolzener Form beim Schmelzpunkt die unter a) genannten aromatischen Isocyanatosulfonsäuren nicht lösenden, einen Schmelzpunkt zwischen 27 und 250°C aufweisenden organischen Feststoffs,

wobei der unter b) genannte Feststoffe gegebenenfalls in inerten Flüssigkeiten gelöst die Partikel der feindispersen Isocyanosulfonsäuren umhüllt.

Als erfindungsgemäß zu stabilisierende aromatische Isocyanatosulfonsäuren eignen sich alle festen aromatischen Isocyanatosulfonsäuren, wie sie bei der Sulfonierung von Mono-, Di- oder Polyisocyanaten in feinteiliger Form anfallen, z.B. die Sulfonierungsprodukte von Phenylisocyanat, p - Tolylisocyanat, p - Chlorphenylisocyanat, p - Nitrophenylisocyanat, p - Methoxyphenylisocyanat, m - Chlorphenylisocyanat, m - Chlormethylphenylisocyanat, p - Chlormethylphenylisocyanate, 4,4' - Stilbendiisocyanat, 4,4' - Dibenzyldiisocyanat, 3,3' - bzw. 2,2' - Dimethyl - 4,4' - diisocyanato-diphenylmethan, 2,5,2', 5' - Tetramethyl - 4,4' - diisocyanato - diphenylmethan, 3,3' - Dimethoxy - 4,4' - diisocyanato - diphenylmethan, 3,3' - Dichlor - 4,4' - diisocyanato - diphenylmethan, 4,4' - Diisocyanato - dimethylmethan, 4,4' - Diisocyanato - diphenylcyclohexylmethan, 4,4' - Diisocyanato - benzophenon, 4,4' - Diisocyanato - diphenylsulfon, 4,4' - Diisocyanato - diphenyläther, 4,4' - Diisocyanato - 3,3' - dibrom - diphenylmethan, 4,4' - Diisocyanato - 3,3' - diäthyl - diphenylmethan, 4,4' - Diisocyanato - diphenyl - äthylen - (1,2), 4,4' - Diisocyanato - diphenyl - sulfid, 1,3- und 1,4 - Phenylendiisocyanat, 2,4- und 2,6 - Toluylendiisocyanat sowie beliebige Gemische dieser Isomeren, Diphenylmethan - 2,4'- und/oder -4,4' - diisocyanat, Naphtylen - 1,5 - diisocyanat, Triphenylmethan - 4,4' - 4'' - triisocyanat, Polyphenyl - polymethylen - polyisocyanate, wie sie durch Anilin - Formaldehyd - Kondensation und anschließende Phosgenierung erhalten und z.B. in den britischen Patentschriften 874 430 und 848 671 beschrieben werden, Carbodiimidgruppen aufweisende Polyisocyanate, wie sie in der deutschen Patentschrift 1 092 007 beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in den deutschen Patentschriften 1022 789, 1 222 067 und 1 027 394, sowie in den deutschen Offenlegungsschriften 1 929 034 und 2 004 048 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394, in der britischen Patentschrift 889 050 und in der französischen Patentschrift 7 017 514 beschrieben werden.

Bevorzugt sind pulverförmige sulfonierte Di- und Triisocyanate, insbesondere die im allgemeinen in Form der Dimeren vorliegenden Mono- und Disulfonsäuren von 4,4' - Diisocyanato - diphenylmethan, 2,4' - Diisocyanato - diphenylmethan, und inbesondere 2,4 - Diisocyanato - toluol und 2,6 - Diisocyanato - toluol sowie den aus diesen Isomeren bestehenden Gemischen. Die Herstellung derartiger pulverförmiger Polyisocyanate ist beispielsweise in US-PS 3 826 769, der Deutschen Patentanmeldung P 25 24 476.2 oder in der Deutschen Patentanmeldung P 26 15 876.9 beschrieben.

Pulverförmige, erfindungsgemäß zu stabilisierende aromatische Isocyanatosulfonsäuren entstehen im allgemeinen dann, wenn man in Analogie zu den in diesen Literaturstellen beschriebenen Verfahrensweisen die Sulfonierung in überschüssigem Isocyanat oder einem inerten organischen Suspendiermittel wie z.B. Dichloräthan oder Tetrachloräthan durchführt. Die Teilchengröße der als feinteilige Pulver anfallenden Isocyanatosulfonsäuren kann darüberhinaus durch geeignete Wahl des Suspendiermittels, die Temperatur bei welcher die Sulfonierung durchgeführt wird und die Rührgeschwindigkeit während der Sulfonierung eingestellt werden. Der Zusatz von Tensiden führt im allgemeinen zu einer Verringerung der Teilchengröße. Die erfindungsgemäß zu stabili-

sierenden aromatischen Isocyanatosulfon-säuren weisen im allgemeinen einen mittleren Teilchendurchmesser zwischen 0,0005 und 0,5 mm auf.

Flüssige, ansulfonierte aromatische Polyisocyanatgemische wie sie z.B. in den Deutschen Offenlegungsschriften 2 227 111, 2 359 614 oder 2 359 615 beschrieben sind, stellen keine erfindungsgemäß zu stabilisierenden Iso-cyanatosulfonsäuren dar.

In den erfindungsgemäßen lagerstabilen Gemischen liegen als zweite, stabilisierend wirkende Komponente inerte organische Feststoffe eines zwischen 27 und 250°C, vorzugsweise zwischen 40 und 120°C liegenden Schmelzpunkt vor. Es handelt sich bei diesen Feststoffen um weitgehend unpolare, mit Wasser nicht mischbare Substanzen, welche in geschmolzener Form beim Schmelzpunkt für die Isocyanatosulfonsäuren keine Lösungsmittel darstellen. Diese Festkörper liegen in den erfindungsgemäßen lagerstabilen Gemischen in Form von die einzelnen Partikel der feindispersen Isocyanatosulfonsäuren umhüllenden Feststoffen oder in Form einer die Isocyanatosulfonsäuren benetzenden Lösung in einem geeigneten Lösungsmittel vor.

Beispiele geeigneter Festkörper eines zwischen 27°C und 250°C vorzugsweise zwischen 40 und 120°C liegenden Schmelzpunkts sind Paraffine, welche insbesondere einen Schmelzbereich zwischen 40 und 95°C aufweisen, feste Halogenparaffine des genannten Schmelzbereichs, feste Äther und Ester von $C_{10}$—$C_{18}$-Alkoholen wie z.B. Octadecyl - methyläther, Di - decyläther, Decyl - acetat, oder Octadecylacetat, Ester und Amide von $C_1$—$C_{18}$-Carbonsäuren wie z.B. Stearinsäure - Äthylester oder N,N - Dimethyl - stearyl - säureamid, Naphthalin, Durol, Diphenylmethan Diphenyl, Terphenyl u.a. feste aromatische Kohlenwasserstoffe, Diphenyläther, Diphenylsulfon, 4,4' - Dichlordiphenylsulfon, natürliche oder synthetische Wachse sowie insbesondere feste Polyester, Polyäther, sowie deren endständig acylierte Derivate. Genannt seien z.B.: Poly-äthylenglykoladipat, Polybutylenglykoladipat, Polybutylenglykol - succinat, Polyäthylen-glykol - sebazat, Polybutylenglykolsebazat, Polyhexandiol - adipat, Polyhexandiol - oxalat, Polyhexandiol - succinat, Polyhexandiol-carbonat, Polybutylenglykoläther, Poly-hexandioläther.

Selbstverständlich können auch Mischester, sowie Äther - Ester aus 3 und mehr der üblichen Glykol- und Carbonsäurekomponenten eingesetzt werden, sofern ihr Schmelzpunkt über 26°C liegt. Vorzugsweise werden solche Polyester bzw. Polyäther eingesetzt, die keine reaktiven Endgruppen mehr aufweisen; deren ursprünglich vorhandene OH-Gruppen beispielsweise durch Umsetzung mit einem Acylierungsmittel wie z.B. Essigsäureanhydrid acyliert bzw. deren ursprünglich vorhandene COOH-Gruppen mit Monoalkoholen wie z.B. Methanol oder Äthanol verestert sind.

Besonders bevorzugt sind ferner Di- bzw. Oligourethane, die aus den genannten Polyestern bzw. Polyäthern durch Umsetzung mit Monoisocyanaten gegebenenfalls unter Zusatz von Diisocyanaten erhalten werden. Als Monoisocyanate eignen sich z.B. Methyl-, Butyl-, Phenyl-, 6-Chlorhexyl- und insbesondere Stearylisocyanat.

Die beste Eignung als Stabilisierungsmittel weisen hydrophobe aber schwach polare Substanzen auf, welche zwar die Isocyanatosulfonsäuren nicht lösen, jedoch im Reaktionsgemisch aus Isocyanatosulfonsäure und Zerwitinoff - aktiver Reaktionskomponente nicht unlöslich sind. Vielmehr sollte sich die schützende inerte Substanz im späteren Reaktionsgemisch, wie es bei der Verwendung der erfindungsgemäß stabilisierten Isocyanatosulfonsäuren beispielsweise bei der Herstellung von Polyurethankunststoffen vorliegt, beim Erwärmen lösen. Aus diesem Grunde sind aliphatische Kohlenwasserstoffe, insbesondere Paraffine erfindungsgemäß weniger bevorzugt.

Sofern die genannten Feststoffe einen Schmelzpunkt von unter 150° aufweisen und die Schmelze hinreichend dünnflüssig ist, können sie allein Verwendung finden. Vorzugsweise werden sie indessen mit inerten Lösungsmitteln zusammen angewendet, wobei die resultierende Lösung mindestens 10% des erfindungsgemäßen Feststoffs enthalten soll.

Falls die erfindungswesentlichen Feststoffe in gelöster Form verwendet werden, kommen als Lösungsmittel Flüssigkeiten in Betracht, die einerseits für die Isocyanatosulfonsäuren keine Lösungsmittel darstellen, die erfindungswesentlichen Festsubstanzen jedoch lösen. Die Flüssigkeiten sind darüber hinaus mit Wasser nicht mischbar. Grundsätzlich sind sowohl derartige Flüssigkeiten mit einem über 90°C vorzugsweise über 110°C liegenden Siedepunkt als auch Flüssigkeiten mit einem unter 90° vorzugsweise unter 60°C liegenden Siedepunkt geeignet. Im Falle der Verwendung der höherseidenden Flüssigkeiten als Lösungs-mittel für die erfindungswesentlichen Fest-körper verbleiben diese Flüssigkeiten im allgemeinen in den erfindungsgemäß stabilisierten Isocyanatosulfonsäuren, so daß die erfindungsgemäßen Substanzen in Form von Feuchtpulvern bzw. Pasten vorliegen, in denen die Isocyanatosulfonsäuren mit der Lösung des erfindungswesentlichen Feststoffs in dem nicht flüchtigen Lösungsmittel benetzt sind. Im Falle der Verwendung der leicht flüchtigen Flüssigkeiten als Lösungsmittel für die erfindungswesentlichen Festkörper verdunsten diese Flüssigkeiten im allgemeinen nach Beendigung des erfindungsgemäßen Verfahrens, so daß die erfindungsgemäßen Gemische ebenso wie im Falle der Verwendung von lösungsmittelfreien Schmelzen der erfindungswesentlichen Feststoffe in Form von mit den erfindungswesentlichen Feststoffen umhüllten Partikeln der Isocyanatosulfonsäuren anfallen.

Es ist jedoch beispielsweise auch möglich, zunächst mit Toluol als Lösungsmittel (Schmelzpunkt: 110°C) ein Feuchtpulver herzustellen und dieses anschließend durch Vakuumbehandlung in ein erfindungsgemäßes trockenes Pulver zu überführen, Beispiele geeigneter Flüssigkeiten eines über 90 vorzugsweise eines über 110°C liegenden Siedepunkts sind Heptan, Octan, Iso-octan, Nonan, Decan, Undecan, Eicosan, sowie Gemische dieser Kohlenwasserstoffe, wie sie z.B. in technischen Benzin- und Paraffinölfraktionen, Testbenzinen sowie in Mineralölen vorliegen; z.B. Solventnaphtha, Waschbenzin, Petroleum, Paraffinöl, polymerisierte Olefine, wie flüssiges Polypropylen und Polybuten, Tetraisobuten, Methylcyclohexan, Cyclododecan, Di-methylcyclohexan, Terpentinöl, Decalin. Weiterhin eignen sich aromatische Kohlenwasserstoffe wie Toluol, o-, m-, p-Xylol, technische Aromatengemische, Cumol, Pseudocumol, Hemellitol, p-Cymol, Tetramethylbenzole, Diisopropylbenzole, isododecylbenzol, Tetralin. Auch Halogen - kohlenwasserstoffe, Ketone, Ester und Äther kommen grundsätzlich in Betracht, sofern sie hinreichend apolar sind, d.h. die Isocyanatsulfonsäure nicht lösen. Dies ist im allgemeinen dann der Fall, wenn der reine Kohlenwasserstoffanteil des Suspendiermittels mindestens 70 Gew.-% beträgt. Beispiele sind: Dibutyläther, Di-sec. butyläther, Diisoamyläther Methyl - isobutyl-keton, 4-Heptanon, 5 - Methyl - 3 - heptanon, 2 - Undecanon, Dinonylketon, Dioctylphthalat, Trioctylphosphat, Dioctyladipat.

Eine weitere Gruppe geeigneter Substanzen sind Perchlorkohlenwasserstoffe, z.B. hochchlorierte Paraffine sowie Chlorbenzol, Dichlorbenzol Chlorcyclohexan.

Beispiele geeigneter leicht flüchtiger Flüssigkeiten sind Petroläther, Hexan, Methylenchlorid, Chloroform, 1,2 - Dichloräthan, Perchloräthylen, Aceton, Methyläthylketon, Äthylacetat oder Cyclohexan. Polare Lösungsmittel wie die letztgenannten Ketone und Ester können nur im Gemisch mit hinreichend apolaren Lösungsmitteln oder Feststoffen Verwendung finden, so daß gewährleistet ist, daß die zu stabilisierenden Isocyanatosulfonsäuren sich in den Lösungen nicht lösen.

Wie aus den beispielhaften Aufzählungen bereits ersichtlich sind im Rahmen der vorliegenden Erfindung unter "inerten" Flüssigkeiten und Festkörpern solche zu verstehen, welche gegenüber Sulfonsäuregruppen und gegenüber Isocyanatgruppen keinerlei Reaktionsbereitschaft zeigen. Flüssigkeiten und gelöst oder als Schmelze zur Anwendung gelangende Festkörper, die diesen Voraussetzungen und den oben definierten Bedingungen entsprechen, sind für die besonders bevorzugte Ausführungsform b) des erfindungsgemäßen Verfahrens geeignet.

Flüssigkeiten und in Form von Lösungen bzw. Schmelzen zur Anwendung gelangende Festkörper, welche gemäß Ausführungsform a) des erfindungsgemäßen Verfahrens eingesetzt werden müssen darüber hinaus auch gegenüber den Sulfonierungsmitteln unter den angewandten Reaktionsbedingungen inert sein. Die meisten beispielhaft genannten Flüssigkeiten und Festkörper sind gegenüber Sulfonierungsmitteln inert, können also ohne weiteres auch als Lösungsmittel für die Sulfonierung gemäß Ausführungsform a) eingesetzt werden. Aromatische Kohlenwasserstoffe sind bekanntermaßen prinzipiell selbst sulfonierbar. Ihre Verwendbarkeit ist keinen Einschränkungen unterworfen, wenn sie gemäß Ausführungsform b) im Anschluß an die Sulfonierung dem Reaktionsgemisch zugesetzt werden. Dagegen ist ihre Einsatz als Lösungsmittel für die zu sulfonierenden Isocyanate nur bedingt möglich, nämlich dann, wenn sie erheblich langsamer mit Sulfonierungsmitteln reagieren also die zu sulfonierenden Isocyanate, oder wenn eine geringfügige Mitsulfonierung dieser Kohlenwasserstoffe nicht stört. So können z.B. Chlorbenzol und Dichlorbenzol als Lösungsmittel für Sulfonierungen eingesetzt werden, dagegen werden Toluol, Xylol, Cumol vorzugsweise nach der Sulfonierung zugesetzt. Bei der Herstellung von Isocyanatopolysulfonsäuren (Derivate mit mehr als einer Sulfonsäuregruppe pro Molekül an zur Sulfonierung eingesetztem Ausgangsisocyanat) muß berücksichtigt werden, daß schon die Disulfonierung erheblich langsamer und schwieriger verläuft als die Monosulfonierung. In diesen Fällen sollten daher bei der Sulfonierung nur gegenüber Schwefeltrioxid inerte Lösungsmittel bzw. Festkörper Anwendung finden. Die erfindungsgemäße Stabilisierung erfolgt im Falle der genannten Polysulfon-säuren im übrigen unabhängig von der Art des verwendeten erfindungsgemäßen Stabilisierrungsmittels, vorzugsweise erst nach der Sulfonierung und nach Entfernen von überschüssigem Sulfonierungsmittel gemäß Ausführungsform b).

Die Durchführung des erfindungsgemäßen Verfahrens zur Stabilisierung der Isocyanato - sulfonsäuren kann nach 2 Varianten erfolgen:

Man kann beispielsweise die Sulfonierung des Isocyanats wie in den oben angegebenen Literaturstellen beschrieben durchführen, wobei jedoch die erfindungswesentliche Flüssigkeit (Schmelze oder Lösung des Feststoffs) vor oder während der Sulfonierung dem zu sulfonierenden Isocyanat zugesetzt wird. Nach Beendigung der Sulfonierungsreaktion kann dann gegebenenfalls ein Teil des eingesetzten Zusatzmittels beispielsweise durch Filtrieren oder Absaugen im Vakuum entfernt werden. Wesentlich bei dieser Variante ist lediglich, daß noch eine solche Menge an erfindungswesentlichem, stabilisierendem inertem Feststoff im Sulfonierungsprodukt verbleibt, der den Mengenverhältnissen der Komponenten in den erfindungsgemäßen Gemischen entspricht.

Man kann auch die Sulfonierung beispielsweise wie in den obengenannten Literaturstellen beschrieben in Abwesenheit von erfindungswesentlichen Stabilisatoren durchführen und das gegebenenfalls noch feuchte, einen Restgehalt an Hilfslösungsmittel wie Dichlorathan oder einen Restgehalt an unsulfoniertem flüssigen Ausgangsisocyanat enthaltende Produkt mit dem erfindungswesentlichen Suspendiermittel vermischen. Wesentlich ist, daß das sulfonierte Isocyanat möglichst unmittelbar nach seiner Herstellung vollständig von dem erfindungswesentlichen Suspendiermittel umhüllt bzw. durchdrungen wird, ohne jedoch darin gelöst zu werden.

Je nach Menge der in den erfindungsgemäßen Gemischen vorliegenden Hilfsflüssigkeit stellen die erfindungsgemäßen Gemische Trockenpulver oder Feuchtpulver oder Pasten dar. Die Menge des erfindungswesentlichen Feststoffs, d.h. bei der erstgenannten Variante des im Gemisch verbleibenden Farbstoffs, wird im allgemeinen so bemessen, daß Gemische entstehen, welche 40—95 Gew.-%, vorzugsweise 60—90 Gew.-%, an aromatischen Isocyanatosulfonsäure und 60—5 Gew.-%, vorzugsweise 10 bis 40 Gew.-% an erfindungswesentlichem, stabilisierendem Feststoff enthalten. Der mittlere Teilchendurchmesser der feindispersen Isocyanatosulfonsäuren beträgt 0,5—500 Mikron.

Die erfindungsgemäßen stabilisierten Zubereitungen können noch Additive enthalten, wodurch die stabilisierende Wirkung erhöht wird oder zusätzliche Stabilisierungseffekte, z.B. gegenüber Vergilbung, erreicht werden können. Hierzu zählen neben den genannten Flüssigkeiten die dem Fachman bekannten Lichtschutzmittel, wie sterisch gehinderte Phenole, UV-Absorber, sowie nichtsulfonierte übliche Polyisocyanate, insbesondere aliphatische Polyisocyanate mit niedrigem Dampfdruck, ferner Organopolysiloxane und Chlorfluorkohlenstoffoele. Die stabilisierten Zubereitungen sind lager- und versandstabil und eignen sich zur Herstellung von Polyurethanen der Verschiedensten Art, z.B. Elastomeren, Schaumstoffen, Beschichtungen, Formkörpern, Klebstoffen. Während Kohlenwasserstoffe wie Octan, Toluol, Xylol bei oder nach der Herstellung bzw. Applikation des Polyurethans verdampfen, bleiben hochsiedende Produkte, wie Phosphate, Phthalate, Polychlorparaffine als Weichmacher oder Mikrotröpfchen im fertigen Polyurethan enthalten.

Die stabilisierten Isocyanatsulfonsäuren sind auch nach längerer Lagerung in Polyestern und Polyäthern sowie in Tetrahydrofuran löslich. Die daraus hergestellten Polyurethane sind frei von Inhomogenitäten und Trübungen.

Ein besonderer Vorteil der erfindungsgemäßen Trockenpulver Pasten und Feuchtpulver ist der verringerte Bedarf an Flüssigkomponenten bei der Herstellung der Reaktionsmischungen bzw. deren verringerte Viskosität bei gegebener Rezeptur. Ferner ist die Menge an bei der Mischung zwangsläufig eingebrachter Luft erheblich geringer, die Mischungen enthalten weniger oder keine Schaumbläschen und liefern homogenere Reaktionsmischungen.

Der besondere Vorteil der erfindungsgemäßen stabilisierten Isocyanatosulfonsäuren ist darin zu sehen, daß die Produkte als trockene Pulver gehandhabt und versandt werden können, ohne daß die Löslichkeit und Reaktionsfähigkeit der Verfahrensprodukte beeinträchtigt wird.

In weiterer Vorteil besteht in der Möglichkeit, die pulverförmigen Isocyanatosulfonsäuren je nach Wunsch dünner oder auch in dickerer Schicht zu umhüllen und damit die Geschwindigkeit der Reaktion in einem Reaktionsmedium zu steuern. Beispielsweise ist es möglich die erfindungsgemäßen Kompositionen auch in wäßrigen Medien zu verteilen, ohne daß momentan Reaktion mit dem Wasser eintritt. Die Tropfzeit hochreaktiver Systeme läßt sich auf diese Weise winschgemäß einstellen.

Beispiel 1

1914 g (11 Mol) Toluylendiisocyanat (Isomerengemisch 2,4:2,6 = 80:20) werden bei 23—30°C im Verlauf von ca. 20 Stunden unter Rühren mit 335 g (4,2 Mol) Schwefeltrioxid umgesetzt, wobei eine dickflüssige Suspension der dimeren Toluylen - diisocyanat - monosulfonsäure im Toluylendiisocyanat entsteht. Das Schwefeltrioxid wird mittels eines schwachen Stickstoffstroms aus erwärmtem 65%igem Oleum freigesetzt und gasförmig mit Stickstoff verdünnt auf die Oberfläche des gerührten Isocyanates aufgeleitet. Die erhaltene Suspension wird mit 500 ml Toluol verdünnt, abgesaugt, und der feste Rückstand 2 mal mit 500 ml einer 20-gew.-%igen Lösung von Diphenyl in Toluol angeschlammt und abgesaugt. Das Toluolfeuchte Produkt wird abgefüllt. Ausbeute 1310 g, Gehalt an Toluol und Diphenyl 26%, Trockensubstanz 970 g, entsprechend 91% der Theorie.

Das Produkt ist ein schwach feuchtes Pulver, das sich ohne zu stauben sehr gut handhaben laßt. Es ist leicht ab- und umzufüllen, backt nicht zusammen und klebt nicht am Spatel.

Beispiel 2

300 g des gemäß Beispiel 1 erhaltenen Feuchtpulvers wird im Vakuumtrockenschrank bei 35° getrocknet und das trockene Pulver abgefüllt. Ausbeute 246 g. Das Produkt ist trocken, frei fließend und staubt nicht. Eine Probe des 6 Wochen gelagerten Produkts ist in Tetrahydrofuran klar löslich.

Beispiel 3

Es wird gemäß Beispiel 1 verfahren, jedoch wird anstelle der 20-proz. Lösung von Diphenyl in Toluol reiner Diphenyläther in geschmolzener Form bei 35° eingesetzt. Die Suspension der dimeren Toluylendiisocyanatmonosulfonsäure in Diphenyläther wird auf einer 40° warmen Nutsche scharf abgesaugft und anschließend unter Kühlung der Filterkuchen zerkleinert. Das Produkt ist auch nach längerer Lagerung an der Luft in Tetrahydrofuran löslich.

### Beispiel 4

Es wird gemäß Beispiel 1 verfahrenm jedoch wird anstelle der 20-proz. Lösung von Diphenyl in Toluol eine 10-proz. Lösung des Umsetzungsprodukts von 1 Mol Polyäthylenglykoladipat (endständige OH-Gruppen, Molekulargewicht 2000) mit 2 Mol Stearylisocyanat in Methylenchlorid eingesetzt. Die gebildete Suspension wird abgesaugt und der Filterkuchen bei Raumtemperatur an der Luft getrocknet. Das trockene Pulver ist nach 4 Wochen Lagerung in Tetrahydrofuran löslich, während eine mit Dichlormethan allein hergestellte und entsprechend behandelte Vergleichsprobe eine trübe Lösung in Tetrahydrofuran liefert.

### Beispiel 5

Es wird gemäß Beispiel 1 verfahren, jedoch wird eine 25-proz. Lösung des Umsetzungsprodukts von 1 Mol Polytetrahydrofuran (Molekulargewicht 3000) mit 2 Mol Chlorhexylisocyanat eingesetzt. Aufarbeitung und Löslichkeitsverhalten wie in Beispiel 4. Beim Einbringen des trockenen Produkts in Wasser tritt erst nach einigen Minuten Gasentwicklung ein.

### Patentansprüche

1. Verfahren zur Erhöhung der Lagerstabilität von feindispersen, festen, durch Sulfonierung von aromatischen Isocyanaten erhaltenen, aromatischen Isocyanatosulfonsäuren, dadurch gekennzeichnet, daß

a) man die Sulfonierung der aromatischen Isocyanate in Gegenwart einer inerten Flüssigkeit durchführt, welche für die aromatischen Isocyanatosolfonsäuren kein Lösungsmittel ist, und welche entweder aus einer Schmelze eines zwischen 27 und 250°C schmelzenden inerten organischen Feststoffs oder aus einer mindestens 10-gew.-%igen Lösung eines derartigen Feststoffs in einem inerten Lösungsmittel besteht, wobei man nach Beendigung der Sulfonierungsreaktion den besagten Feststoff zumindestens teilweise in dem Sulfonierungsprodukt beläßt, oder

b) man in Abwesenheit einer Flüssigkeit der unter a) definierten Art hergestellte, feste, feindisperse, aromatische Isocyanatosulfonsäuren mit einer derartigen Flüssigkeit möglichst bald nach der Herstellung vermengt.

2. Lagerstabiles, gegebenenfalls in Form eines Feuchtpulvers oder einer Paste vorliegendes Gemisch enthaltend

a) 40—95 Gew.-% an festen, feindispersen, aromatischen Isocyanatosulfonsäuren und

b) 60—5 Gew.-% eines unpolaren, gegenüber Isocyanatgruppen und Solfonsäuregruppen inerten, die unter a) genannten aromatischen Isocyanatosulfonsäuren nicht lösenden, einen Schmelzpunkt zwischen 27 und 250°C aufweisenden organischen Feststoffs,

wobei der unter b) genannte Feststoff gegebenenfalls in inerten Flüssigkeiten gelöst die Partikel der feindispersen Isocyanatosulfonsäuren umhüllt.

### Revendications

1. Procédé pour accroître la stabilité au stockage d'acides isocyanatosulfoniques aromatiques solides finement dispersés, obtenus par sulfonation d'isocyanates aromatiques, ce procédé se caractérisant en ce que

a) on procède à la sulfonation des isocyanates aromatiques en présence d'un liquide inerte qui n'est pas solvant des acides isocyanatosulfoniques aromatiques et qui consiste soit en la masse fondue d'une substance solide organique inerte fondant entre 27 et 250°C, soit en une solution à au moins 10% en poids d'une telle substance solide dans un solvant inerte et, après la fin de la réaction de sulfonation, on laisse ladite substance solide, en partie au moins, dans le produit de sulfonation, ou bien

b) on mélange, aussitôt que possible après leur préparation, les acides isocyanatosulfoniques aromatiques solides finement dispersés, préparés en l'absence d'un liquide tel que défini ci-dessus sous a), avec un tel liquide.

2. Mélanges stables à la conservation, éventuellement à l'état de poudre humide ou de pâte, contenant

a) 40 à 95% en poids d'acides isocyanatosulfoniques aromatiques solides finement dispersés et

b) 60 à 5% en poids d'une substance solide organique non polaire, inerte à l'égard des groupes isocyanate et des groupes acide, sulfonique, ne dissolvant pas les acides isocyanatosulfoniques aromatiques mentionnés sous a) et présentant un point de fusion compris entre 27 et 250°C,

la substance solide mentionnée ci-dessus sous b), éventuellement dissoute dans des liquides inertes, enrobant les particules des acides isocyanatosulfoniques finement dispersés.

### Claims

1. Process for increasing the stability in storage of finely dispersed solid aromatic isocyanatosulphonic acids obtained by sulphonation of aromatic isocyanates, characterised in that

a) sulphonation of the aromatic isocyanates is carried out in the presence of an inert liquid which is a non-solvent for the aromatic isocyanatosulphonic acids and which consists either of a melt of an inert organic solid which melts at between 27° and 250°C or of an at least 10% by weight solution of such a solid in an inert solvent, the said solid being at least partly left in the sulphonation product after termination of the sulphonation reaction, or

b) solid, finely dispersed aromatic isocyanatosulphonic acids prepared in the absence of a liquid of the type defined under a) are mixed with such a liquid, as soon as possible after they have been prepared.

2. Mixture which is stable in storage and which is optionally in the form of a moist powder or paste, containing

a) 40—95% by weight of solid, finely dispersed, aromatic isocyanatosulphonic acids and

b) 60—5% by weight of an apolar organic solid substance which is inert towards isocyanate groups and sulphonic acid groups, has a melting point between 27° and 250°C and is a non-solvent for the aromatic isocyanatosulphonic acids mentioned under a),

the solid substance mentioned under b), optionally dissolved in inert liquids, enveloping the particles of the finely dispersed isocyanatosulphonic acids.